# EUROPÄISCHE PATENTSCHRIFT

(11) **EP 1 531 882 B1**
(45) Veröffentlichungstag und Bekanntmachung des Hinweises auf die Patenterteilung: **31.12.2008**
(21) Anmeldenummer: 03783917.2
(22) Anmeldetag: 10.07.2003
(51) Int. Cl.: A61M 1/00

(54) **CHIRURGISCHE EINRICHTUNG ZUR ENTNAHME VON GEWEBEZELLEN AUS EINER BIOLOGISCHEN STRUKTUR INSBESONDERE ZUR LIPOSUKTION**
SURGICAL DEVICE FOR REMOVING TISSUE CELLS FROM A BIOLOGICAL STRUCTURE ESPECIALLY FOR LIPOSUCTION
DISPOSITIF CHIRURGICAL POUR PRELEVER DES CELLULES DE TISSUS DANS UNE STRUCTURE BIOLOGIQUE

(30) Priorität: 12.07.2002 DE 20211555 U
(43) Veröffentlichungstag der Anmeldung: 25.05.2005
(73) Patentinhaber: Human Med AG, 19061 Schwerin (DE)
(72) Erfinder: Pein, Andreas, 23911 Einhaus (DE)
(74) Vertreter: Jaap, Reinhard
(86) Internationale Anmeldenummer: PCT/DE2003/002321
(87) Internationale Veröffentlichungsnummer: WO 2004/014460

(56) Entgegenhaltungen:
- WO-A-01/91827
- DE-A- 10 033 278
- DE-U- 20 009 786
- US-A- 5 254 106
- US-A- 5 766 194

## Beschreibung

Die Erfindung bezieht sich auf eine chirurgische Einrichtung nach *den Oberbegriffen der Ansprüche 1 und 3.*

Derartige Einrichtungen werden in chirurgischen Kliniken zu kosmetischen Zwecken und zu Zwecken der Behandlung von Krankheiten, aber auch zur Gewinnung vermehrungsfähiger Gewebezellen eingesetzt.

Es ist allgemein bekannt, zum Beispiel überschüssige Fettgewebezellen zum Zwecke der Kosmetik abzusaugen, in dem in einem ersten Arbeitsschritt eine Arbeitsflüssigkeit unter Druck in das Fettgewebe eingespritzt wird, bei dem das Fettgewebe durch eine chemische Reaktion mit der Arbeitsflüssigkeit aufgelöst wird, und in einem zweiten Arbeitsschritt eine unter der Kraft eines Unterdruckes stehende Absaugkanüle in das entsprechende Fettgewebe eingeschoben wird, bei dem die Saugkraft das Fettgewebe endgültig aus dem Gewebeverband herausreist und das Gemisch aus gelöstem Fettgewebe und Arbeitsflüssigkeit abtransportiert. Dieses Gemisch wird in einen Aufnahmebehälter aufgefangen und anschließend entsorgt. Dabei ist die Absaugkanüle so ausgebildet, dass sie mehrere gleichmäßig am Umfang verteile Absaugbohrungen besitzt.

Aus der US-A 5.968.008 ist nun eine solche Fettabsaugvorrichtung bekannt, die mit einer innerhalb der Absaugkanüle angeordneten Einspritzleitung ausgerüstet ist. Die Einspritzleitung mündet in eine Austrittsbohrung, aus der ein runder Flüssigkeitsstrahl austritt. Mit dieser Vorrichtung werden die beiden Arbeitsschritte des Einspritzens von Arbeitsflüssigkeit und des Absaugens von Fettgewebe mit der Arbeitsflüssigkeit zeitlich überlagert, wodurch der chirurgische Eingriff zeitsparender und kontinuierlicher wird.

In der DE 200 09 786 U1 *und in der* WOl01l91827 A1 ist nun *jeweils* eine gleichartige Fettabsaugvorrichtung beschrieben, deren Einspritzleitung mit einer schlitzartigen Austrittsöffnung ausgerüstet ist, aus der der Strahl der Arbeitsflüssigkeit fächerartig austritt. Dieser fächerartige Flüssigkeitsstrahl soll die Arbeitsflüssigkeit besser verteilen, um einen gleichförmigen Abtrag eines größeren Volumens an Fettgewebe zu ermöglichen.

Alle diese genannten Fettabsaugvorrichtungen sind also darauf ausgerichtet, das Fettgewebe im Zusammenwirken zwischen der Lösungskraft der Arbeitsflüssigkeit und der Kraft des Saugstromes aus dem Gewebeverband herauszureißen. Und gerade dieses Zusammenwirken zwischen beiden Kraftkomponenten ist problematisch, da die Saugkraft mit einer konstanten Größe ausgestattet ist und der Lösungsprozess des Arbeitsmittels zeitabhängig abläuft. Diese nichtvorhandene Abstimmung der Kräfte untereinander führt dazu, dass die konstante Saugkraft zu Beginn des zeitlich ablaufenden Lösungsprozesses zu gering ist, weil noch nicht ausreichend Gewebezellen abgelöst sind, und am Ende des ablaufenden Lösungsprozesses zu groß ist, weil die Gewebezellen nach Beendigung des Lösungsprozesses inzwischen freiliegen. Damit sind die Gewebezellen entweder einer zu langen Einflusszeit der Arbeitsflüssigkeit oder einer zu großen Saugkraft ausgesetzt. In beiden Fällen kommt es zur Zerstörung von abzusaugenden und auch von erhaltenswerten Gewebezellen. Das belastet den menschlichen Körper und kompliziert und verlängert den Heilungsprozess.
Die abgesaugten Fettgewebezellen sind dann auch nicht mehr für eine Weiterverwendung geeignet, da die Fettgewebezellen durch den Absaugvorgang und durch den zerstörenden Einfluss der Arbeitsflüssigkeit zerstört sind.

In der DE 100 33 278 A1 der Anmelderin wurde erstmals eine Einrichtung zur Entnahme von Gewebezellen aus einer biologischen Struktur beschrieben, die primär darauf abgestellt ist, die überflüssigen Fettgewebezellen mit Hilfe eines unter Druck stehenden Flüssigkeitsstrahles vollständig von den benachbarten Gewebezellen zu trennen. Dazu ist die Austrittsöffnung der Einspritzleitung so ausgeführt, dass ein Flachstrahl mit einer vorderen Schneidkante ausgebildet wird, die wie ein Schabegerät arbeitet und die so eine schälende Wirkung auf die Fettgewebezellen erzielt. Die Flüssigkeit ist chemisch neutral und steht unter einem solchen Druck, der im Zusammenspiel mit den glatten und weichen Fettgewebezellen in intelligenter Weise zwischen benachbarte Gewebezellen eindringt und sie auseinander drückt und dabei die festen und die gewebezellenzusammenhaltenden Sehnen mechanisch abtrennt, ohne die Gewebezellen zu zerstören. Die so in schonender Weise abgetrennten Gewebezellen werden zusammen mit der neutralen Flüssigkeit mit einer relativ geringen Saugkraft abgesaugt und entsorgt oder von der neutralen Flüssigkeit wieder getrennt und weiterverwendet. Der besondere Vorteil dieser chirurgischen Einrichtung liegt darin, dass die Fettgewebezellen allein durch die Kraft des Trennstrahles abgetrennt und die Absaugung der Fettgewebezellen zusammen mit der neutralen Flüssigkeit durch die Kraft des Saugstromes erfolgt. Dadurch gibt es im Gegensatz zum Stand der Technik keine notwendige Abstimmung zwischen der Trennkraft und der Saugkraft. So können sowohl die Trennkraft als auch die Saugkraft unabhängig voneinander jeweils auf die für eine schonende Behandlung erforderliche Größe ausgewählt werden. Im Gegensatz zu den Vorrichtungen nach dem Stand der Technik, wo eine von Blut durchtränkte Flüssigkeit entsteht, zeigt die neue chirurgische Entnahmeeinrichtung einen von den Fettgewebezellen geprägten milchigweißen Absaugstrom.

Es hat sich aber gezeigt, dass auch mit dieser chirurgischen Entnahmeeinrichtung noch körperliche Belastungen auftreten und noch ein bestimmter Anteil von Fettgewebezellen beschädigt wird.

Der Erfindung liegt daher die Aufgabe zu Grunde, die *Schnitteigenschaften des Flüssigkeitsstrahls zu verbessern.*
Diese Aufgabe wird durch die kennzeichnenden Merkmale *der Ansprüche* 1 *und 3* gelöst. Weitere Ausgestaltungsmöglichkeiten ergeben sich aus den Unteransprüchen 2 *und 4.*

Die neue chirurgische Einrichtung zeichnet sich insbesondere dadurch aus, dass mit einer sehr geringen Trennkraft und mit einer sehr geringen Absaugkraft gearbeitet werden kann. Das schont im erheblichen Maße den menschlichen Körper während des chirurgischen Eingriffs und es schont die Gewebezellen, sodass diese für andere Zwecke weiterverwendet werden können.

Die sehr geringe Trennkraft ist dadurch zu erklären, dass nicht wie bisher mit einer frontalen Trennkante gearbeitet wird, der naturgemäß wegen ihrer Breite viel Widerstand entgegengebracht wird und die dadurch eine hohe Kraft aufbringen muss. Vielmehr dringt bei der neuen Einrichtung zunächst die Trennspitze und dann nachfolgend die schräg stehenden Trennkanten in die Zwischenräume der Gewebezellen ein, sodass die zu trennenden Gewebeteile nicht mehr abgeschlagen, sondern entlang der Trennkanten abgeschnitten werden. Dieser reine Schneidenvorgang ist relativ widerstandsarm, sodass auch die Schneid- bzw. Trennkraft klein gehalten werden kann. Die erforderliche Schneidkraft kann in vorteilhafter Weise durch die Wahl des Winkels a und damit dem Schneidenwinkel der Trennkante ausgewählt werden.

Auch die erforderliche Absaugkraft kann sehr gering gehalten werden, was in einfacher Weise so zu erklären ist. Da die Trennkante grundsätzlich den Absaugbohrungen vorgelagert ist und die Trennkräfte in Strömungsrichtung, also weg von den Absaugbohrungen, gerichtet sind, haben auch alle die Gewebeteile, die von den Trennkräften belastet werden, das Bestreben, sich von den Absaugbohrungen zu entfernen. Um diese sich wegbewegenden Gewebeteile abzusaugen, müssen sie erst abgebremst, in ihrer Richtung umgedreht und dann wieder beschleunigt werden. Somit muss die Saugkraft sowohl den Trennkräften als auch den Massenkräften der Gewebeteile entgegenwirken, was innerhalb des menschlichen Körpers ein komplizierter Bewegungsablauf ist. Da die neue chirurgische Einrichtung wegen ihrer neuartigen Ausrichtung des flachen Flüssigkcitsstrahles eine geringere Trennkraft benötigt, ist auch der Rückholvorgang der Gewebeteile kraftschonender.

Diese neue chirurgische Einrichtung macht es wegen der geringen Trennkraft und der geringen Absaugkraft in vorteilhafter Weise möglich, den Düsenschlitz V-förmig auszubilden, sodass sehr breite Trennkanten entstehen. Das erhöht die Effektivität der chirurgischen Einrichtung. Es ist auch zweckmäßig, mehr als einen separaten flachen Flüssigkeitsstrahl einzusetzen, was in effektiver Weise ebenfalls den Aktionsradius erweitert.

Es liegt dem Fachmann nahe, auf der Grundlage der neuen technischen Lehre weitere Ausführungsformen auszuwählen.

Die Erfindung soll anhand mehrerer Ausführungsbeispiele näher erläutert werden.
Dazu zeigen:
- Fig. 1:: das Operationshandstück mit einem gegenüber der Achse zweifach abgewinkelten Flachstrahl in einer ersten Ansicht,
- Fig. 2:: das Operationshandstück nach Fig. 1 in einer anderen Ansicht,
- Fig. 3:: die Einzelheit X aus den Fig. 1 und 2,
- Fig. 4:: das Operationshandstück mit einem gegenüber der Achse einfach abgewinkelten Winkelstrahl in einer Ansicht,
- Fig. 5:: die Einzelheit X nach der Fig. 4,
- Fig. 6:: das Operationshandstück nach Fig. 5 mit einer um 180° gedrehten Ausrichtung,
- Fig. 7:: die Einzelheit X nach der Fig. 6,
- Fig. 8:: das Operationshandstück mit zwei gegenüber der Achse abgewinkelten Flachstrahlen, die winklig zueinander ausgerichtet sind,
- Fig. 9:: die Einzelheit X nach der Fig. 8;
- Fig. 10:: das Operationshandstück nach der Fig. 8 mit einer um 180° gedrehten Ausrichtung,
- Fig. 11:: die Einzelheit X nach der Fig. 10,
- Fig. 12:: das Operationshandstück mit zwei gegenüber der Achse zweifach abgewinkelten Flachstrahlen, die zueinander zu einem Flügelrad ausgerichtet sind, in einer Ansicht,
- Fig. 13:: das Flügelrad in einer anderen Ansicht und
- Fig. 14:: die Einzelheit X nach der Fig. 12.

Die chirurgische Einrichtung zur Entnahme von vitalen Gewebezellen aus einer biologischen Struktur besteht aus einer Flüssigkeitstrenneinrichtung zum Trennen einer biologischen Struktur, wie sie beispielsweise aus der EP 0 551920 B1 der Anmelderin bekannt ist, und einer Absaugeinrichtung. Sowohl die Flüssigkeitstrenneinrichtung als auch die Absaugeinrichtung sind allgemein bekannt und brauchen daher nicht gezeigt zu werden. Die Flüssigkeitstrenneinrichtung besteht demnach aus einem Vorratsbehälter, einer Druckpumpe und einer Einspritzleitung und die Absaugeinrichtung besitzt einen Auffangbehälter, eine Saugpumpe und eine Absaugleitung. Die Einspritzleitung der Flüssigkeitstrenneinrichtung und die Absaugleitung der Absaugeinrichtung münden gemeinsam in ein Operationshandstück 1.

Die verwendeten Figuren zeigen das distale Ende dieses Operationshandstückes 1. Dieses Ende des Operationshandstückes 1 besteht aus einem äußeren Absaugrohr 2, das auf der proximalen Seite mit der Saugpumpe der Absaugeinrichtung verbunden ist und das am distalen Ende einen kegeligen Ansatz 3 mit einer mittigen Aufnahmebohrung besitzt. Das Absaugrohr 2 ist mit ein oder mehreren Reihen von radialen Absaugbohrungen 4 ausgerüstet, die am Umfang des Absaugrohres 2 in besonderer Weise angeordnet sind.

Im inneren des Absaugrohres 2 befindet sich eine Einspritzkanüle 5, die auf der proximalen Seite über eine Einspritzleitung mit der Druckpumpe der Flüssigkeitstrenneinrichtung verbunden ist. Die Einspritzkanüle 5 ist mit Spiel in die mittige Aufnahmebohrung des Absaugrohres 2 eingepasst. Dabei ragt die Einspritzkanüle 5 in ihrer Länge um einen bestimmten Betrag aus der Durchgangsbohrung des Absaugrohres 2 heraus. Das distale Ende der Einspritzkanüle 5 ist als eine Einspritzdüse 6 ausgebildet und besitzt daher eine Kegelspitze 7 mit einem Spitzenwinkel von etwa 90°. In der Kegelmantelfläche der Kegelspitze 7 befinden sich ein oder mehrere Düsenschlitze 8, die in besonderer Weise ausgebildet und angeordnet sind.

Die einzelnen Figuren veranschaulichen unterschiedliche Ausführungsformen dieser besonderen Düsenschlitze 8 in der Einspritzdüse 6 und der besonders angeordneten Absaugbohrungen 4 im Absaugrohr 2.

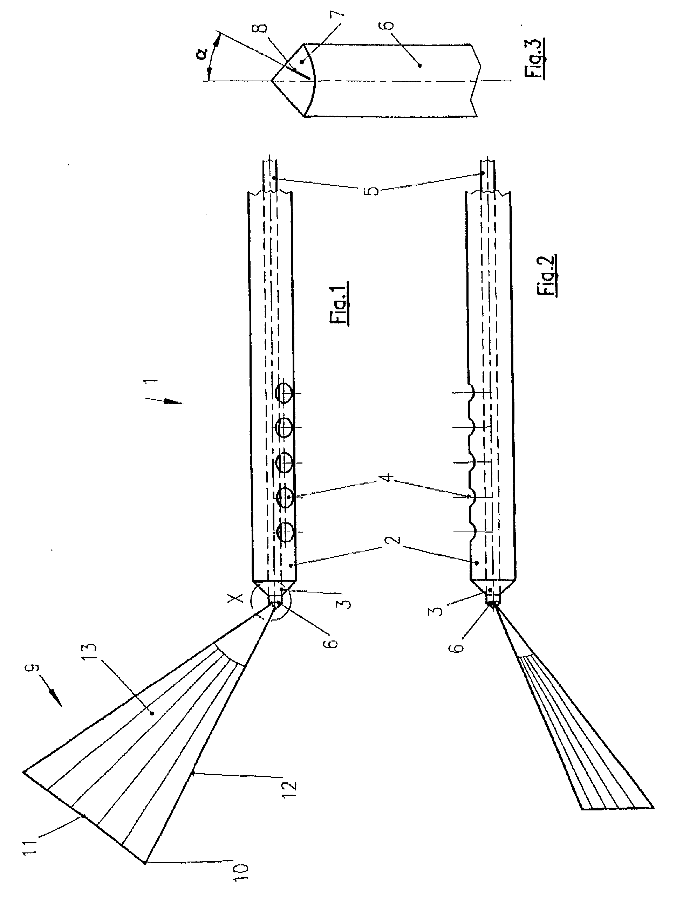

So zeigen die Fig. 1 bis 3 einen Düsenschlitz 8, der gegenüber der axialen Schnittebene der Kegelspitze 7 unter einem Winkel α von maximal 30° geneigt ist und der sich von der Kegelkante des Kegeldurchmessers bis zur Sichtkante der Kegelspitze 7 erstreckt. Damit ergibt sich ein flacher Flüssigkeitsstrahl 9, der gegenüber der Kegelachse zweifach abgewinkelt ist und der so eine vorgelagerte Trennspitze 10 mit einer ersten Trennkante 11 und einer zweiten Trennkante 12 ausbildet. Beide Trennkanten 11 und 12 sind zur Trennspitze 10 benachbart angeordnet. Außerdem bildet sich eine obenliegende Schälfläche 13 aus, an der die abgetrennten Gewebeteile abgleiten und in Richtung der Absaugbohrungen 4 im Absaugrohr 2 geleitet werden. Dazu sind die Absaugbohrungen 4 im Absaugrohr 2 in Richtung der Achse des Absaugrohres 2 auf einer Reihe angeordnet und auf die Seite der Schälfläche 13 und auf die Lage der Trennspitze 10 ausgerichtet.

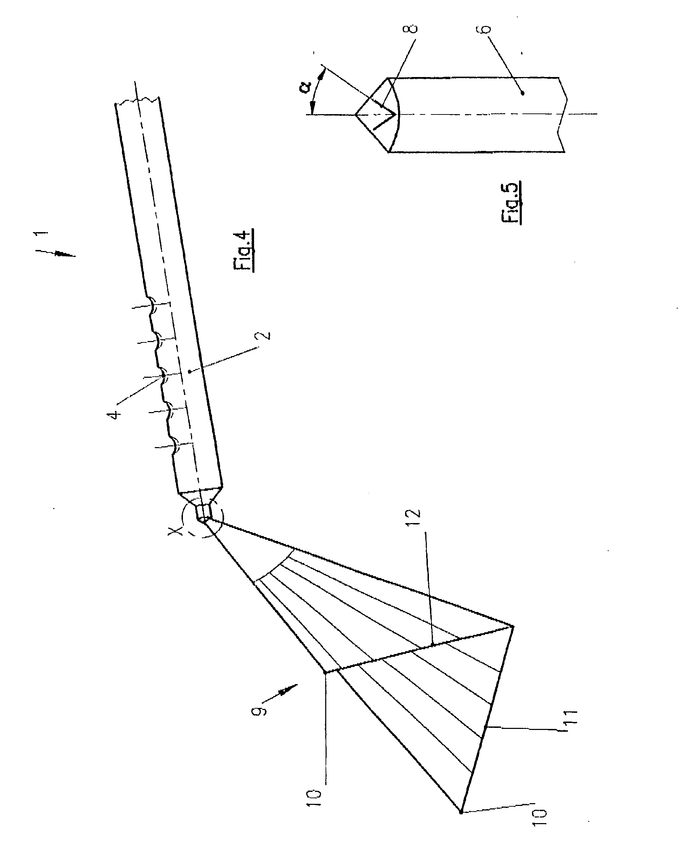

Die Fig. 4 und 5 zeigen einen Düsenschlitz 8, der V-förmig ausgebildet ist und dessen beide Schenkeln von einer gemeinsamen, an der Kegelkante der Kegelspitze 7 liegenden Spitze jeweils bis zur Sichtkante der Kegelspitze 7 verlaufen. Beide Schenkeln des V-förmigen Düsenschlitzes 8 verlaufen unter einem Winkel von etwa 90°. Damit bildet sich auch ein abgewinkelter Flüssigkeitsstrahl 9 mit zwei vorgelagerten Trennspitzen 10 und einer ersten Trennkante 11 und einer zweiten Trennkante 12. Die Schälfläche 13 wird vom Winkel des Trennstrahles 9 eingeschlossen.

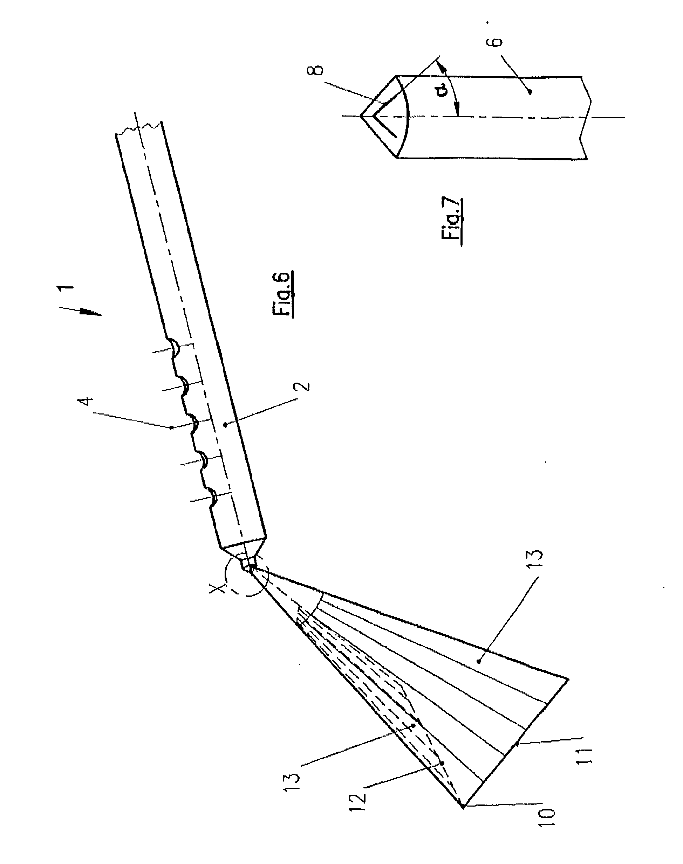

Die Fig. 6 und 7 zeigen einen weiteren abgewinkelten Düsenschlitz 8, der ebenfalls V-förmig ausgebildet, aber seitenverkehrt zum V-förmigen Düsenschlitz 8 der Fig. 4 und 5 angeordnet ist. Damit bilden sich zwei außenliegende Schälflächen 13 aus.

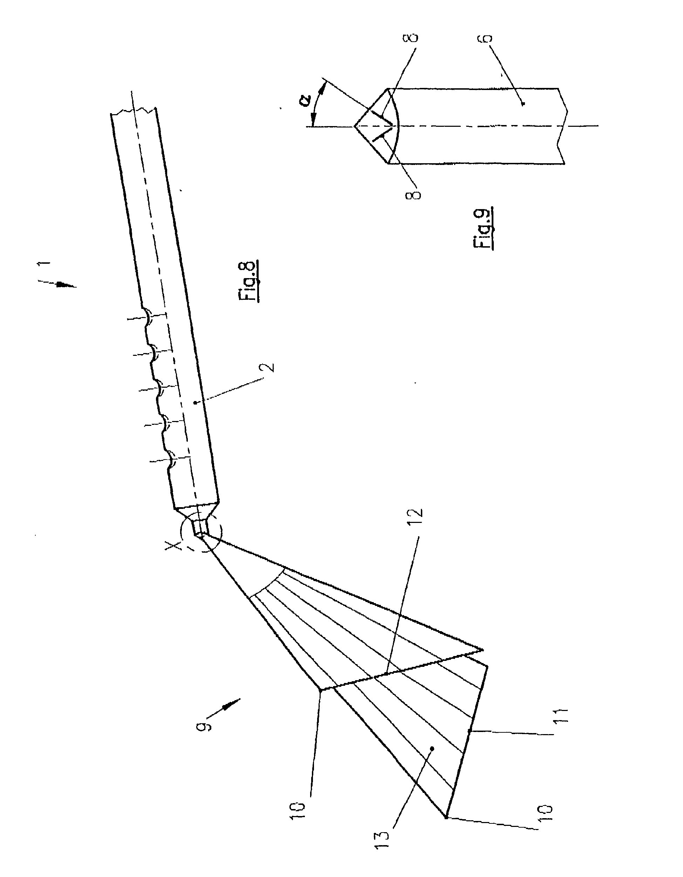

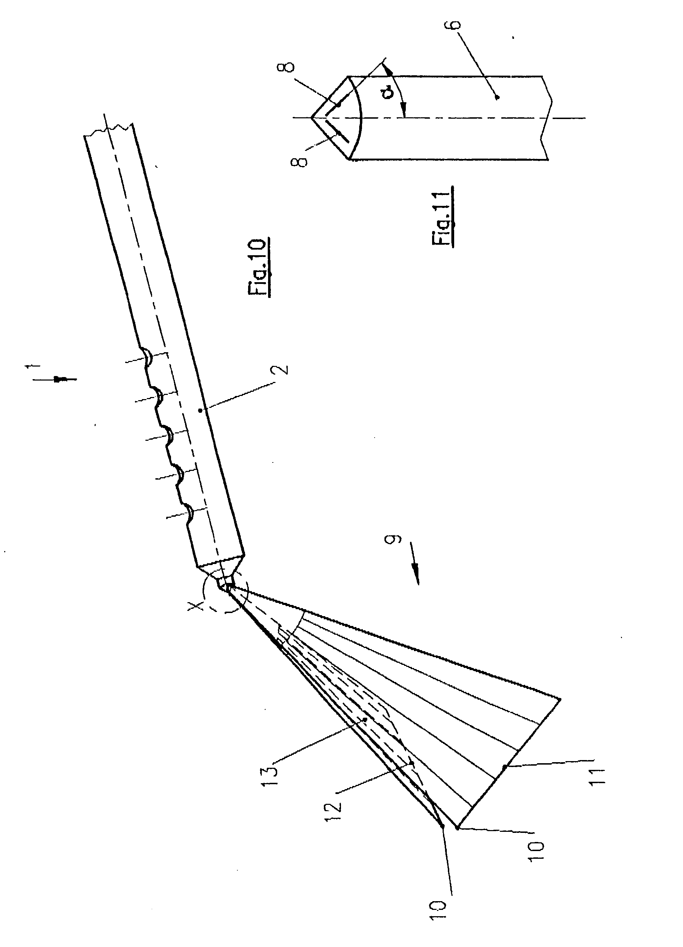

Eine andere Ausführungsform zeigen die Fig. 8 und 9 sowie die Fig. 10 und 11. Hier sind zwei Düsenschlitze 8 V-förmig aber in Abstand zueinander ausgerichtet. So bilden sich in der Ausführung nach Fig. 8, 9 zwei Trennspitzen 10, eine erste Trennkante 11, eine zweite Trennkante 12 sowie zwei innenliegende Schalflächen 13 aus. Durch die andere Ausrichtung der beiden Düsenschlitze 8 nach den Fig. 10, 11 entstehen am Flüssigkeitsstrahl zwei außenliegende Schalflächen 13.

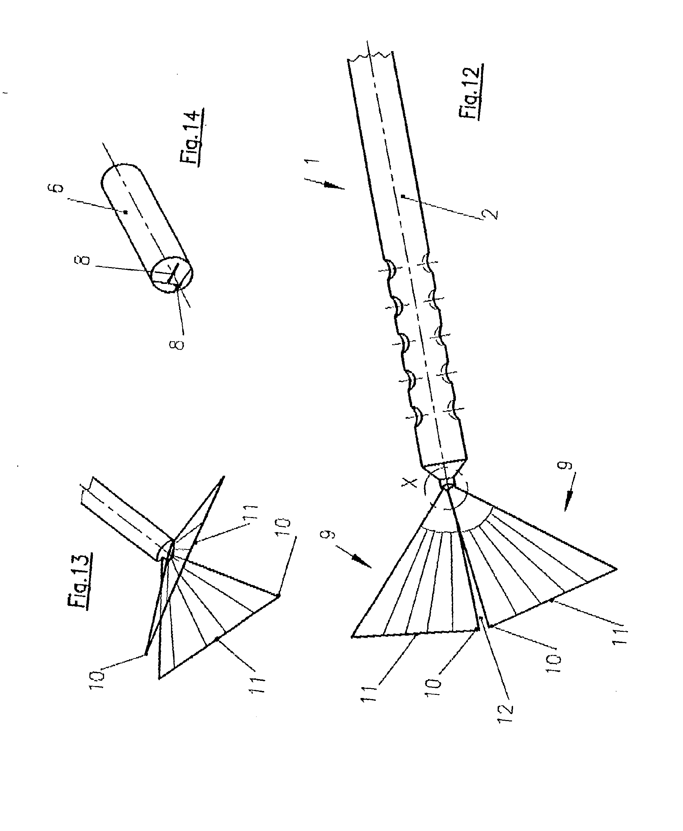

Eine besondere Ausführungsform zeigen die Fig. 12 bis 14. Hier befinden sich zwei separate Düsenschlitze 8 jeweils einerseits und andererseits der Kegelspitze 7, die voneinander wegdriftende Flüssigkeitsstrahlen 9 erzeugen, die zusammen die Form eines Flügelrades annehmen. Jedem Flüssigkeitsstrahl 9 ist eine vorgelagerte Trennspitze 10, eine erste Trennkante 11 und eine zweite Trennkante 12 sowie eine Schälfläche 13 zugeordnet. Dazu ist das Absaugrohr 2 mit zwei Reihen von Absaugbohrungen 4 ausgestattet, von denen jeweils eine Reihe von Absaugbohrungen 4 im funktionellen Zusammenhang mit der Schälfläche 13 eines Flüssigkeitsstrahles 9 steht.

### Liste der Bezugszeichen

- 1: Operationshandstück
- 2: Absaugrohr
- 3: kegeliger Ansatz
- 4: Absaugbohrung
- 5: Einspritzkanüle
- 6: Einspritzdüse
- 7: Kegelspitze
- 8: Düsenschlitz
- 9: Flüssigkeitsstrahl
- 10: Trennspitze
- 11: erste Trennkante
- 12: zweite Trennkante
- 13: Schalfläche

## Patentansprüche

1. Chirurgische Einrichtung zur Entnahme von Gewebezellen aus einer biologischen Struktur, bestehend aus einer Flüssigkeitsstrahleinrichtung zur Erzeugung eines trennfähigen Flüssigkeitsstrahles und einer Absaugeinrichtung zum Absaugen abgetrennter Gewebezellen und der eingestrahlten Flüssigkeit und aus einem mit der Flüssigkeitsstrableinrichtung und mit der Absaugeinrichtung verbundenen Operationshandstück (1), wobei
- das Operationshandstück (1) aus einem äußeren Absaugrohr (2) und einer inneren, das Absaugrohr (2) durchdringenden Einspritzkanüle (5) besteht, die zwischen sich einen ringförmigen Absaugringkanal ausbilden,
- das Absaugrohr (2) ein oder mehrere Reihen von radialen Absaugbohrungen (4) besitzt und
- die Einspritzkanüle (5) an ihrem distalen Ende mit einer Kegelspitze (7) verschlossen und in der Mantelfläche der Kegelspitze (7) mit einem, einen Flachstrahl ausbildenden Düsenschlitz (8) ausgerüstet ist, wobei der Flachstrahl eine Schälfläche (13) ausbildet,
**dadurch gekennzeichnet, dass** mindestens ein Düsenschlitz (8) vorgesehen ist, der sich in der abgewickelten und außerhalb des Düsenschlitzes (8) aufgeschnittenen Mantelfläche der Kegelspitze (7) durchgängig darstellt und der so ausgerichtet ist, dass sich die Enden des Düsenschlitzes (8) ungleich von der Kegelspitze (7) entfernt befinden.

2. Chirurgische Einrichtung nach Anspruch 1,
**dadurch gekennzeichnet, dass** mindestens zwei separate Düsenschlitze (8) auf der Mantelfläche der Kegelspitze (7) angeordnet sind, *wobei die zwei Düsenschlitze (8) V-förmig aber in Abstand* zueinander ausgerichtet sind.

3. Chirurgische Einrichtung zur Entnahme von Gewebezellen aus einer biologischen Struktur, bestehend aus einer Flüssigkeitsstrahleinrichtung zur Erzeugung eines trennfähigen Flüssigkeitsstrahles und einer Absaugeinrichtung zum Absaugen abgetrennter Gewebezellen und der eingestrahlten Flüssigkeit und aus einem mit der Flüssigkeitsstrahleinrichtung und mit der Absaugeinrichtung verbundenen Operationshandstück (1), wobei
- das Operationshandstück (1) aus einem äußeren Absaugrohr (2) und einer inneren, das Absaugrohr (2) durchdringenden Einspritzkanüle (5) besteht, die zwischen sich einen ringförmigen Absaugringkanal ausbilden,
- das Absaugrohr (2) ein oder mehrere Reihen von radialen Absaugbohrungen (4) besitzt und
- die Einspritzkanüle (5) an ihrem distalen Ende mit einer Kegelspitze (7) verschlossen und in der Mantelfläche der Kegelspitze (7) mit einem, einen Flachstrahl ausbildenden Düsenschlitz (8) ausgerüstet ist, wobei der Flachstrahl eine Schälfläche (13) ausbildet,
**dadurch gekennzeichnet, dass** mindestens ein Düsenschlitz (8) vorgesehen ist, der sich in der abgewickelten und außerhalb des Düsenschlitzes (8) aufgeschnittenen Mantelfläche der Kegelspitze (7) durchgängig darstellt, wobei der durchgängige Düsenschlitz (8) V-förmig ausgebildet ist und die von beiden zusammenlaufenden Schenkeln gebildete Spitze des Düsenschlitzes (8) in der axialen Ebene der Kegelspitze (7) angeordnet ist.

4. Chirurgische Einrichtung nach den Ansprüchen 1 oder 3,
**dadurch gekennzeichnet, dass** das Absaugrohr (2) so viele Reihen von Absaugbohrungen (4) hat, wie Schälflächen (13) vorhanden sind und jede Reihe von Absaugbohrungcn auf die Seite der Schälfläche (13) ausgerichtet ist.

## Claims

1. Surgical device for removing tissue cells from a biological structure, comprising a fluid jet device for producing a separable fluid jet and a suction device for extracting separated tissue cells and the fluid which has been introduced, and a manual operating member (1) which is connected to the fluid jet device and the suction device,
- the manual operating member (1) comprising an outer suction pipe (2) and an inner injection cannula (5) which extends through the suction pipe (2), together forming an annular suction ring channel,
- the suction pipe (2) having one or more rows of radial suction holes (4) and
- the injection cannula (5) being closed at the distal end thereof with a conical tip (7) and being provided, in the outer face of the conical tip (7), with a nozzle slot (8) which forms a flat jet, the flat jet forming a separator surface (13),
**characterised in that** there is provided at least one nozzle slot (8) which is continuous in the developed outer face of the conical tip (7) opening outside the nozzle slot (8), and which is orientated in such a manner that the ends of the nozzle slot (8) are located at different distances from the conical tip (7).

2. Surgical device according to claim 1,
**characterised in that** at least two separate nozzle slots (8) are arranged on the outer face of the conical tip (7), the two nozzle slots (8) being V-shaped but being orientated with spacing from each other.

3. Surgical device for removing tissue cells from a biological structure, comprising a fluid jet device for producing a separable fluid jet and a suction device for extracting separated tissue cells and the fluid which has been introduced, and a manual operating member (1) which is connected to the fluid jet device and the suction device,
- the manual operating member (1) comprising an outer suction pipe (2) and an inner injection cannula (5) which extends through the suction pipe (2), together forming an annular suction ring channel,
- the suction pipe (2) having one or more rows of radial suction holes (4) and
- the injection cannula (5) being closed at the distal end thereof with a conical tip (7) and being provided, in the outer face of the conical tip (7), with a nozzle slot (8) which forms a flat jet, the flat jet forming a separator surface (13),
**characterised in that** there is provided at least one nozzle slot (8) which is continuous in the developed outer face of the conical tip (7) opening outside the nozzle slot (8), the continuous nozzle slot (8) being constructed in a V-shaped manner and the tip of the nozzle slot (8) that is formed by the two converging members being arranged in the axial plane of the conical tip (7).

4. Surgical device according to claims 1 or 3,
**characterised in that** the suction pipe (2) has as many rows of suction holes (4) as there are separator surfaces (13) and each row of suction holes is orientated towards the side of the separator surface (13).

## Revendications

1. Dispositif chirurgical pour prélever des cellules de tissus dans une structure biologique et constitué d'un dispositif pour produire un jet de liquide de séparation et d'un dispositif pour aspirer des cellules de tissus séparées et le liquide projeté, le dispositif comportant en outre une pièce manuelle opérationnelle (1) reliée au dispositif à jet de liquide et au dispositif d'aspiration, dans lequel
- la pièce manuelle opérationnelle (1) est constituée d'un tube d'aspiration extérieur (2) et d'une canule d'injection intérieure (5) pénétrant dans le tube d'aspiration (2), ces deux éléments définissant entre eux un canal d'aspiration annulaire,
- le tube d'aspiration (2) est équipé d'une ou de plusieurs rangées de perçages d'aspiration radiaux (4), et
- la canule d'injection (5) est fermée à son extrémité distale par une pointe conique (7), pourvue sur sa surface enveloppe d'une fente de buse (8) formant un jet plat définissant une surface de séparation (13),
**caractérisé en ce qu'**
il est prévu au moins une fente de buse (8) découpée sur l'extérieur de la surface enveloppe de la pointe conique (7), et
la fente de buse (8) est orientée de sorte que ses extrémités ne soient pas situées à la même distance de la pointe conique (7).

2. Dispositif chirurgical selon la revendication 1,
**caractérisé en ce qu'**
il est prévu au moins deux fentes de buse (8) séparées sur la surface enveloppe de la pointe conique (7), ces deux fentes (8) étant en forme de V mais orientées à distance l'une de l'autre.

3. Dispositif chirurgical pour prélever des cellules de tissus dans une structure biologique, constitué d'un dispositif pour produire un jet de liquide de séparation et d'un dispositif pour aspirer des cellules de tissus séparées et le liquide projeté, le dispositif comportant en outre une pièce manuelle opérationnelle (1) reliée au dispositif à jet de liquide et au dispositif d'aspiration, dans lequel
- la pièce manuelle opérationnelle (1) est constituée d'un tube d'aspiration extérieur (2) et d'une canule d'injection intérieure (5) pénétrant dans le tube d'aspiration (2), ces deux éléments définissant entre eux un canal d'aspiration annulaire,
- le tube d'aspiration (2) est équipé d'une ou de plusieurs rangées de perçages d'aspiration radiaux (4), et
- la canule d'injection (5) est fermée à son extrémité distale par une pointe conique (7) pourvue sur sa surface enveloppe d'une fente de buse (8) formant un jet plat, définissant une surface de séparation (13),
**caractérisé en ce qu'**
il est prévu au moins une fente de buse (8) découpée sur l'extérieur de la surface enveloppe de la pointe conique (7), et
la pointe des deux branches convergentes de la fente de buse (8) est située dans le plan axial de la pointe conique (7).

4. Dispositif chirurgical selon la revendication 1 ou 3,
**caractérisé en ce que**
le tube d'aspiration (2) est équipé d'un nombre de rangées de perçages d'aspiration (4) égal au nombre de surfaces de séparation (13) et chaque rangée de perçages d'aspiration est orientée du côté de la surface de séparation (13).
